(19) Europäisches Patentamt
European Patent Office
Office européen des brevets

(11) Veröffentlichungsnummer: **0 374 759**
**A2**

# (12) EUROPÄISCHE PATENTANMELDUNG

(21) Anmeldenummer: **89123246.4**

(51) Int. Cl.5: **C07H 15/252, A61K 31/70**

(22) Anmeldetag: **15.12.89**

(30) Priorität: **20.12.88 DE 3842836**

(43) Veröffentlichungstag der Anmeldung:
**27.06.90 Patentblatt 90/26**

(84) Benannte Vertragsstaaten:
**AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kolar, Cenek, Dr.**
**Deutschhausstrasse 20**
**D-3550 Marburg(DE)**
Erfinder: **Kraemer, Hans Peter, Dr.**
**Birkenweg 16**
**D-3550 Marburg(DE)**
Erfinder: **Dehmel, Konrad**
**Blumengarten 11**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

## (54) Rhodomycine mit einer modifizierten Kohlenhydrat-Einheit.

(57) Es werden 7-O-Glycosyl-rhodomycine, die der nachfolgenden allgemeinen Formel I

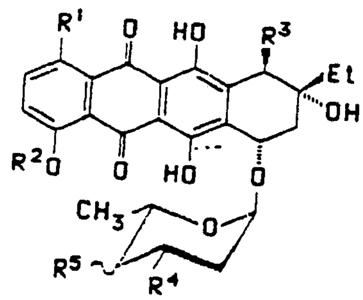

Formel I

entsprechen, beschrieben, worin die Reste folgende Bedeutung haben:
$R^1$ ist ein Wasserstoffatom oder eine Hydroxygruppe,
$R^2$ ist ein Wasserstoffatom oder eine $C_1$-$C_4$-Alkylgruppe,
$R^3$ ist eine Hydroxygruppe, eine Acylschutzgruppe oder Methyloxycarbonylgruppe,
$R^4$ ist ein Wasserstoffatom, eine O-Acylschutzgruppe, eine Azidogruppe, eine Amino- oder Trifluoroacetylaminogruppe, eine Di-$C_1$-$C_4$-alkylaminogruppe oder Cyanomethylaminogruppe und
$R^5$ ist eine Azidogruppe, Amino- oder Trifluoroacetylaminogruppe, eine Di-$C_1$-$C_4$-alkylaminogruppe oder Cyanomethylaminogruppe,
wobei Acylschutzgruppe eine Acetyl-, Mono-, Di- oder Trihalogenacetylgruppe- mit Fluor oder Chlor als Halogen oder die p-Nitrobenzoylgruppe bedeutet,
und ein Verfahren zu ihrer Herstellung sowie ihre Verwendung als Arzneimittel.

EP 0 374 759 A2

## Rhodomycine mit einer modifizierten Kohlenhydrat-Einheit

Die vorliegende Erfindung bezieht sich auf neue zytostatisch wirksame Anthracyclin-Derivate, und sie betrifft speziell 7-0-Glycosyl-rhodomycine, die am C-4′-Atom der Kohlenhydrat-Einheit modifiziert sind, Verfahren zu ihrer Herstellung sowie ihre Verwendung als Arzneimittel.

Die Substanzklasse der Anthracycline ist in der Fachliteratur eingehend beschrieben. Doxorubicin und sein 14-Desoxyanalogon Daunorubicin werden als die erfolgreichsten Vertreter dieser Substanzklasse genannt, die in der Klinik zur Behandlung einer großen Anzahl von festen Tumoren und Leukämien eingesetzt werden. Der Erfolg dieser speziellen Verbindungen ist jedoch nicht bei allen Patienten gleich, und die Erfolgsrate ist bei einigen speziellen Tumorarten wie Dickdarmkrebs und Melanom geringer. Nebenwirkungen der Behandlung mit Doxorubicin und Daunorubicin sind unter anderen eine Schädigung des Kreislaufsystems und dafür charakteristische Beschwerden.

Eine Anzahl weiterer Analoga, die sowohl in dem Aglyconteil als auch in der Kohlenhydrat-Einheit modifiziert wurden, ist ferner beschrieben, vor allem solche des Doxorubicin-Daunorubicin-Types. Bei den Rhodomycinen wurden Derivate beschrieben, die ein natürliches oder synthetisches 3-Aminozucker-Segment enthalten, aber Verbindungen, die am C-4′-Atom der Kohlenhydrat-Einheit modifiziert sind, wurden bisher nicht beschrieben.

Ausgehend von diesem Stand der Technik ist es die Aufgabe der vorliegenden Erfindung, ausgehend von einem Rhodomycin-Aglycon und einem C-4-modifizierten funktionalisierten Kohlenhydrat neue Rhodomycinonglycoside zu schaffen, die sich durch ein neues Wirkungsspektrum und eine geringere Toxizität auszeichnen.

Überraschenderweise hat es sich bei der Glycosidierung von 10-Acyl geschützten ß-rhodomycinon mit einem 4-Amino-Daunosamin-Derivat ergeben, daß nur alpha-0-glycosidisch verknüpfte Produkte entstehen. Die alpha-0-glycosidische Bindung in den Anthracyclinen ist für die Entfaltung ihrer zytostatischen Wirksamkeit essentiell. Die neuen Derivate waren weniger zytotoxisch als das bekannte 7-0-(Daunosaminyl)-ß-rhodomycinon.

Aufbauend auf diesen Erkenntnissen hat es sich die vorliegende Erfindung weiterhin zur Aufgabe gestellt, ausgehend vom Rhodomycin-Aglycon und 4-Amino- oder Azido-Kohlenhydrat-Derivaten, 7-0-Glycosyl-rhodomycinone herzustellen, die ein verbessertes Wirkungsspektrum haben und als tumor-therapeutische Mittel angewandt werden können.

Gelöst wird diese Aufgabe mit zytostatisch wirksamen Anthracyclin-Derivaten, die der Formel I

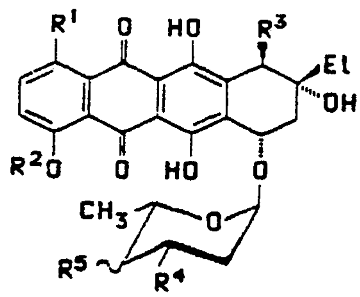

Formel I

entsprechen, worin die Reste folgende Bedeutung haben:

$R^1$ ist ein Wasserstoffatom oder eine Hydroxygruppe,

$R^2$ ist ein Wasserstoffatom oder eine $C_1$-$C_4$-Alkylgruppe,

$R^3$ ist eine Hydroxygruppe, eine O-Acylschutzgruppe oder die Methyloxycarbonylgruppe,

$R^4$ ist ein Wasserstoffatom, eine O-Acylschutzgruppe, eine Azidogruppe, eine Amino- oder Trifluoroacetylaminogruppe, eine Di-$C_1$-$C_4$-alkylaminogruppe oder Cyanomethylaminogruppe und

$R^5$ ist eine Azidogruppe, Amino- oder Trifluoroacetylaminogruppe, eine Di-$C_1$-$C_4$-alkylaminogruppe oder Cyanomethylaminogruppe,

wobei Acylschutzgruppe eine Acetyl-, Mono-, Di- oder Trihalogenacetylgruppe mit Fluor oder Chlor als Halogen oder die p-Nitrobenzoylgruppe bedeutet.

Bevorzugt sind Verbindungen der Formel I, worin

$R^1$ H oder OH,

$R^2$ H oder $CH_3$,

$R^3$ OH, $F_3CCOO$, p-$NO_2$-PhCOO oder $COOCH_3$,
$R^4$ H, OH,p-$NO_2$-PhCOO, $N_3$, $NH_2$, $NHCOCF_3$, $N(CH_3)_2$ oder $NHCH_2CN$ und
$R^5$ $N_3$, $NH_2$, $NHCOCF_3$, $N(CH_3)_2$ oder $NHCH_2$-CN sind.

Die Verbindungen der Formel I können gegebenenfalls als Ammoniumsalze vorliegen.

Gegenstand der Erfindung ist weiterhin ein Verfahren zur Herstellung einer der Verbindungen der Formel I, dadurch gekennzeichnet, daß man

a) eine Aglycon-Verbindung der Formel II

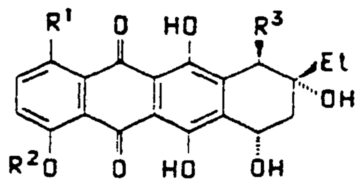

Formel II

worin die Reste
$R^1$ H oder OH,
$R^2$ H oder $C_1$-$C_4$-Alkyl und
$R^3$ O-Acylschutzgruppe oder $COOCH_3$ sind,
mit einem funktionalisierten Desoxyzucker der Formel III oder IV,

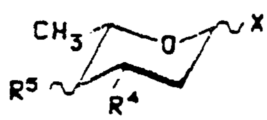

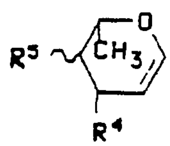

Formel III Formel IV

worin die Reste
$R^4$ ein Wasserstoffatom, eine O-Acylschutzgruppe, Azidogruppe oder Trifluoroacetylaminogruppe,
$R^5$ eine Azidogruppe oder eine Trifluoroacetylaminogruppe und
X eine Acetyloxy-, p-Nitrobenzoyloxygruppe oder ein Chloratom darstellen,
in Gegenwart eines Katalysators vorzugsweise eines Trifluoromethansulfonsäure-tri-$C_1$-$C_4$-alkylsilylesters oder dem Silbersalz der Trifluoromethansulfonsäure zu einem 7-0-Glycosyl-rhodomycinon-Derivat umsetzt, und in dem Produkt die Acylschutzgruppen partiell oder vollständig alkali-hydrolytisch abspaltet, und die in dem Produkt vorhandene Azidogruppe hydrogenolytisch in Gegenwart eines Hydrierungskatalysators wie Palladium/Kohle in die Aminogruppe überführt, wobei eine Verbindung der Formel I entsteht,
worin die Reste
$R^1$ und $R^2$ unverändert bleiben, und
$R^3$ OH oder $COOCH_3$,
$R^4$ ein Wasserstoffatom, eine Hydroxygruppe, eine Acetyloxy-, Trifluoroacetyloxy- oder p-Nitrobenzoyloxygruppe oder eine Azido-, Trifluoroacetylamino-oder Aminogruppe, und
$R^5$ eine Azidogruppe, eine Amino- oder Trifluoroacetylaminogruppe darstellen,

b) eine einen Aminozucker enthaltende Verbindung der Formel I aus dem ersten Verfahrensschritt a) in an sich bekannter Weise unter den Bedingungen der Alkylierung mit einem Halogenessigsäurenitril oder der reduktiven Alkylierung mit einem $C_1$-$C_4$-Aldehyd in Gegenwart eines Alkalicyanoborhydrides zu einer weiteren Verbindung der Formel I umsetzt,
in der die Reste
$R^1$, $R^2$ und $R^3$ unverändert sind, und
$R^4$ eine Di-$C_1$-$C_4$-alkylaminogruppe oder Cyanomethylaminogruppe und
$R^5$ eine Di-$C_1$-$C_4$-alkylaminogruppe oder Cyanomethylaminogruppe darstellen.

Die Verbindungen der Formel I können gegebenenfalls in Ammoniumsalze von pharmazeutisch unbedenklichen anorganischen oder organischen Säuren überführt werden. Folgende Säuren sind hier stellvertretend genannt: Salzsäure, Glutaminsäure und Glucuronsäure.

Gegenstand der Erfindung ist weiterhin die Verwendung einer Verbindung der Formel I als Arzneimittel.

Die Erfindung betrifft weiterhin pharmazeutische Zubereitungen, welche ein Anthracyclinglycosid der Formel I oder eines seiner pharmazeutisch annehmbaren Salze zusammen mit einem pharmazeutisch annehmbaren Verdünnungsmittel oder Träger enthalten. Diese Zubereitungen enthalten eine therapeutisch wirksame Menge des Anthracyclinglycosides oder seines Salzes.

Die Erfindung betrifft weiterhin die Verwendung der Anthracyclinglycoside der Formel I oder ihrer Salze in einem Verfahren zur Herstellung eines Mittels zur Behandlung bestimmter Säugetiertumore durch Verabreichung einer therapeutisch wirksamen Menge an einen Patienten.

Die Bestimmung der zytostatischen Wirksamkeit der hier beschriebenen Verbindungen erfolgte in vitro an L1210-Leukämiezellen der Maus oder in vivo an L1210-Leukämie, B-16-Melanoma und Lewis Lung Adenocarzinoma. Die akute Toxizität der Verbindungen wurde an NMRI-Mäusen ermittelt. Die Methoden sowie Ergebnisse dieser Untersuchung sind in dem experimentellen Teil beschrieben.

## Beispiele

Die Struktur der in den folgenden Beispielen beschriebenen Verbindungen wurde mittels NMR- und MS-Analytik ermittelt. Der Verlauf der Reaktionen sowie die chemische Reinheit der Verbindungen wurde dünnschichtchromatographisch oder HPLC-mäßig untersucht.

Zur Herstellung der erfindungsgemäßen 7-O-Glycosyl-rhodomycinon-Verbindungen wurden folgende Rhodomycinon-Aglycone als Ausgangsverbindungen verwendet:

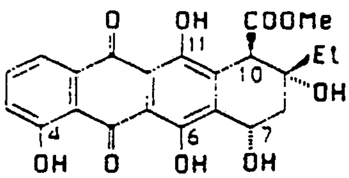

epsilon-Isorhodomycinon (Verbindung 1)

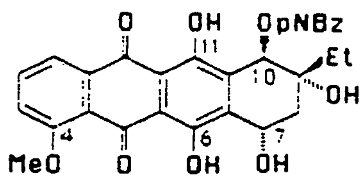

4-O-Methyl-10-O-p-nitrobenzoyl-ß-rhodomycinon (Verbindung 2)

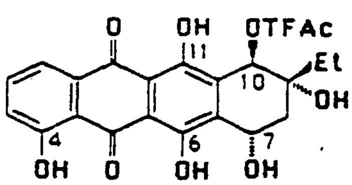

10-O-Trifluoroacetyl-ß-rhodomycinon (Verbindung 3)

Die Rhodomycinon-Aglycone wurden nach den in der Anthracyclinchemie üblichen Verfahren hergestellt.

Die zur Glycosidierung der Aglycone verwendeten funktionalisierten Kohlenhydrate wurden in Analogie zu den in der Kohlenhydratchemie üblichen Verfahren hergestellt. C. Monneret, J. Boivin, A. Martin und M.

Pais in Anthracycline Antibiotics, Edit. H.S. El Khadem, 1982, S. 225 - 251 sowie D. Horton und W. Priebe, ibid., S. 197 - 224.

Beispiel 1

Herstellung von epsilon-Isorhodomycinen (Glycosidierungs- und Entblockierungsreaktionen)

7-O-(3′-O-p-Nitrobenzoyl-2′,4′,6′-tridesoxy-4′-trifluoro-acetamido-alpha-L-lyxohexopyranosyl)-epsilon-isorhodomycinon (Verbindung 4)

200 mg (0.45 mmol) Verbindung 1 wurden in 40 ml Dichlormethan/Aceton (10:1) gelöst und mit 680 mg (2.8 äq) 1,3-Bis-O-(p-nitrobenzoyl)-2,4,6-tridesoxy-4-trifluoroacetamido-L-lyxohexopyranose und 400 mg Molekularsieb 4 Angstrom versetzt. Zu der auf -30° C gekühlten Reaktionsmischung wurden unter Feuchtigkeitsausschluß 0.35 ml (5 äq) Trifluormethansulfonsäure-trimethylsilylester zugegeben. Der Reaktionsansatz wurde anschließend 2 Stunden bei -30° C gerührt, dann mit 0.62 ml (10 äq) Triethylamin neutralisiert und abfiltriert. Das Filtrat wurde dreimal mit Eiswasser ausgewaschen. Die organische Phase wurde über Natriumsulfat getrocknet und in Vakuum eingedampft. Das erhaltene Rohprodukt (500 mg), von dem ein kleiner Teil zwecks Strukturermittlung auf einer Kieselgelplatte gereinigt wurde, wurde in die folgende Reaktion eingesetzt.

7-O-(2′,4′,6′-Tridesoxy-4′-trifluoroacetamido-alpha-L-lyxo-hexopyranosyl)-epsilon-isorhodomycinon (Verbindung 5)

500 mg Verbindung 4 (Rohprodukt) wurden in 40 ml Chloroform/Methanol gelöst und mit 1 ml 0.1 n wäßriger NaOH-Lösung bei Raumtemperatur versetzt. Der Reaktionsansatz wurde 2 Stunden gerührt und anschließend mit 1 ml 0.1 n wäßriger Salzsäure neutralisiert. Anschließend wurde der Ansatz dreimal mit Eiswasser unter Rückextraktion mit Dichlormethan ausgewaschen. Die organische Phase wurde über Natriumsulfat getrocknet und in Vakuum eingedampft. Der erhaltene Rückstand wurde säulenchromatographisch (Kieselgel; Dichlormethan/Methanol 20:1) gereinigt.
Ausbeute: 240 mg (79 %)

7-O-(4′-Amino-2′,4′,6′-tridesoxy-alpha-L-lyxohexopyranosyl)-epsilon-isorhodomycinon (Verbindung 5)

200 mg (0.298 mmol) Verbindung 5 wurden in 30 ml Chloroform/Methanol gelöst und mit 1 n wäßriger NaOH-Lösung versetzt. Nach 2 Stunden Rühren bei Raumtemperatur wurde der Reaktionsansatz mit 1 ml wäßriger Salzsäure neutralisiert und in Vakuum eingedampft. Der Rückstand wurde in 25 ml Chloroform/Methanol aufgenommen und zum Trocknen mit Natriumsulfat verrührt. Nach Abfiltrieren des Trocknungsmittels wurde das Filtrat eingedampft. Der Rückstand wurde säulenchromatographisch (Kieselgel; Chloroform/Methanol 5:1) gereinigt.
Ausbeute: 130 mg (76 %)
MS: FAB m/e = 574 $(M+H^+)$

Beispiel 2

Herstellung von 4-O-Methyl-ß-rhodomycinen (Glycosidierungs- und Entblockierungsreaktionen)

4-O-Methyl-10-O-p-nitrobenzoyl-7-O-(3′-O-p-nitrobenzoyl-2′,4′,6′-tridesoxy-4′-trifluoroacetamido-alpha-L-lyxohexopyranosyl)-ß-rhodomycinon (Verbindung 6)

1.5 g (2.73 mmol) Verbindung 2 wurden in 300 ml Dichlormethan/Aceton (10:1) gelöst und mit 2.95 g (2

äq) 1,3-Bis-O-(p-nitrobenzoyl)-2,4,6-tridesoxy-4-trifluoroacetamido-L-lyxohexopyranose und 3 g Molekularsieb 4 A versetzt. Zu der auf -30° C abgekühlten Reaktionsmischung wurden 2.1 ml (5 äq) Trifluormethansulfonsäure-trimethylsilylester zugegeben, und der Ansatz wurde unter Feuchtigkeitsausschluß 1 Stunde gerührt. Die Reaktionsmischung wurde mit 3.7 ml (10 äq) Triethylamin versetzt und abfiltriert. Das Filtrat wurde dreimal mit Wasser ausgewaschen, über Natriumsulfat getrocknet und in Vakuum eingedampft. Das erhaltene Rohprodukt wurde säulenchromatographisch (Kieselgel; Dichlormethan/Petrolether/Essigsäureethylester/Aceton 70:25:2.5:2.5) gereinigt.
Ausbeute: 1.74 g (70 %)
(alpha)$_D$ = +304° (c = 0.05 in Chloroform)
$^1$H-NMR und H,H-COSY (300 MHz, $CDCl_3$, delta): 7.94 (dd, J = 7.5 Hz und 1 Hz, H-1), 7.72 (t, J = 7.5 Hz und 8 Hz, H-2), 7.34 (dd, J = 8 Hz und 1 Hz, H-3), 5.27 (d, J = 4 Hz, H-7), 2.15 (dd, J = 15 Hz und 4 Hz, H-8a), 2.38 (d, J = 15 Hz, H-8b), 6.53 (s, H-10), 1.83 (m, J = 15 Hz und 7.4 Hz, H-13a), 1.48 (m, J = 15 Hz und 7.4 Hz, H-13b), 1.04 (t, J = 7.4 Hz, H-14), 13.87 und 13.74 (s, PhOH), 4.02 (s, OMe), 3.55 (s, 9-OH), 7.96-8.20 (m, p-NO2Ph), 5.62 (d, J = 4.5 Hz, H-1′), 1.93 (td, J = 13 Hz, 12.5 Hz und 4.5 Hz, H-2′ax), 2.21 (dd, J = 13 Hz und 5.5 Hz, H-2′eq), 5.32 (ddd, J = 12.5 Hz, 5.5 Hz und 3.5 Hz, H-3′), 4.53 (d, J = 8.5 Hz und 3.5 Hz, H-4′), 4.50 (q, J = 6.5 Hz, H-5′), 1.25 (d, J = 6.5 Hz, H-6′), 6.53 (d, J = 8.5 Hz, N-H)

4-O-Methyl-7-O-(2′,4′,6′-tridesoxy-4′-trifluoro- acetamido-alpha-L-lyxohexopyranosyl)-ß-rhodomycinon (Verbindung 7)

174 mg (0.19 mmol) Verbindung 6 wurden in 30 ml Chloroform/ Methanol gelöst und mit 1 ml 1 n NaOH-LÖsung verrührt. Nach 30 min wurde die Reaktion durch die Zugabe von 1 ml 1 n Salzsäure abgebrochen. Die in Vakuum eingedampfte Reaktionsmischung wurde säulenchromatographisch (Kieselgel; Chloroform/Methanol 5:1) gereinigt.
Ausbeute: 78 mg (66 %)
(alpha)$_D$ = +445° (c = 0.1 in Chloroform)

4-O-Methyl-7-O-(4′-amino-2′,4, 6′-tridesoxy-alpha-L-lyxohexo-pyranosyl)-ß-rhodomycinon (Verbindung 8)

0.923 g (1 mmol) Verbindung 6 wurden in 150 ml Chloroform/ Methanol gelöst und mit 12 ml 1 n wäßriger NaOH-Lösung versetzt. Nach 3 Stunden Rühren bei Raumtemperatur wurde der Reaktionsansatz mit 12 ml 1 n Salzsäure neutralisiert und in Vakuum eingedampft. Der in Chloroform/Methanol (5:1) gelöste Rückstand wurde über Natriumsulfat getrocknet. Dann wurde abfiltriert, und das Filtrat wurde in Vakuum eingedampft. Das resultierende Rohprodukt wurde säulenchromatographisch über Kieselgel (Laufmittel: Chloroform/Methanol/Ammoniak 65:35:1) gereinigt.
Ausbeute: 396 mg (75 %)
MS: FAB m/e = 530 ($M+H^+$)
(alpha)$_D$ = +41° (c = 0.1 in Chloroform)
$^1$H-NMR, H,H-COSY (300 MHz, $CDCl_3$/MeOD 5:1, delta): 7.82 (dd, J = 7.5 Hz und 1 Hz, H-1), 7.64 (t, J = 8 Hz und 7.5 Hz, H-2), 7.26 (dd, J = 8 Hz und 1 Hz, H-3), 4.98 (br.s, J = 3.8 Hz und 2 Hz, H-7), 2.11 (d, J = 15 Hz und 2 Hz, H-8a), 2.04 (dd, J = 15 Hz und 3.8 Hz, H-8b), 4.74 (s, H-10), 1.76 (m, J = 15 Hz und 7.5 Hz, H-13a), 1.69 (m, J = 15 Hz und 7.5 Hz, H-13b), 1.01 (t, J = 7.5 Hz, H-14), 3.95 (s, OMe), 5.35 (d, J = 3.7 Hz, H-1′), 1.61 (ddd, J = 13 Hz, 13 Hz und 3.7 Hz, H-2ax), 1.78 (dd, J = 13 Hz und 4.0 Hz, H-2′eq), 3.77 (m, J = 13 Hz, 4.0 Hz und 3.5 Hz, H-3′), 2.82 (d, J = 3.5 Hz, H-4′), 4.12 (q, J = 6.5 Hz, H-5′), 1.23 (d, J = 6.5 Hz, H-6′)

4-O-Methyl-10-O-p-nitrobenzoyl-7-O-(2′,3′,4′,6′-tetradesoxy-3′,4′-bis-(trifluoroacetamido)-alpha-L-lyxohexopyranosyl)-ß-rhodomycinon (Verbindung 9)

1 g (1.82 mmol) Verbindung 2 wurden in 200 ml Dichlormethan/Aceton (10:1) gelöst und unter Rühren mit 2.21 g (2.5 äq) 3,4-Bis-(trifluoracetamido)-1-O-p-nitrobenzoyl-2,3,4,6-tetradesoxy-alpha,ß-L-lyxohexopyranose und 2 g Molekularsieb 4 A versetzt. Die Suspension wurde auf -30° C abgekühlt, und es wurden 1.4 ml (5 äq) Trifluoromethansulfonsäure-trimethylsilylester zugegeben. Nach 2 Stunden Rühren bei -30° C wurde das Reaktionsgemisch mit 2.5 ml (10 äq) Triethylamin versetzt und anschließend abfiltriert. Das Filtrat wurde dreimal mit Wasser ausgewaschen, dann über Natriumsulfat getrocknet und in Vakuum

eingedampft. Das resultierende Produkt wurde säulenchromatographisch über Kieselgel (Elutionsmittel: Dichlormethan/ Aceton 15:1) gereinigt.
Ausbeute: 1.34 g (85 %); Schmelzpunkt: 220-223° C
$(alpha)_D$ = +406° (c = 0.5 in Chloroform)

4-O-Methyl-7-O-(3′,4′-diamino-2′,3′,4′,6′-tetradesoxy-alpha-L-lyxohexopyranosyl)-ß-rhodomycinon (Verbindung 10)

1.34 g (1.54 mmol) Verbindung 9 wurden in 200 ml Chloroform/Methanol gelöst und bei Raumtemperatur mit 60 ml 1 n NaOH versetzt. Nach 3 Stunden wurde das Reaktionsgemisch mit 60 ml 1 n HCl neutralisiert und in Vakuum eingedampft. Der Rückstand wurde in Chloroform/Methanol (5:1) gelöst, und nach der Zugabe von Natriumsulfat wurde die Suspension 20 min gerührt. Es wurde abfiltriert und in Vakuum eingedampft. Der Rückstand wurde säulenchromatographisch über Kieselgel (Elutionsmittel: Chloroform/Methanol/Ammoniak 65:35:1) gereinigt.
Ausbeute: 0.63 g (77 %)
MS: FAB, m/e = 529 $(M+H^+)$
(alpha)D = +355° (c = 0.2 in Methanol)
$^1$H-NMR, H,H-COSY (300 MHz, MeOD, delta): 7.50 (dd, J = 7.5 Hz und 1 Hz, H-1), 7.44 (t, J = 8.5 Hz und 7.5 Hz, H-2), 7.15 (dd, J = 8.5 Hz und 1 Hz, H-3), 4.93 (dd, J = 4 Hz und 1.5 Hz, H-7), 2.06 (dd, J = 15 Hz und 4 Hz, H-8a), 2.14 (dd, J = 15 Hz und 1.5 Hz, H-8b), 4.68 (s, H-10), 1.68 (m, J = 15 Hz und 7.5 Hz, H-13a), 1.74 (m, J = 15 Hz und 7.5 Hz, H-13b), 1.06 (t, J = 7.5 Hz, H-14), 3.78 (s, OMe), 5.32 (d, J = 3 Hz, H-1′), 1.64 (ddd, J = 13 Hz, 13 Hz und H-2′ax), 1.76 (dd, J = 13 Hz und 4 Hz, H-2′eq), 3.06 (t, J = 13 Hz, 4 Hz und 3 Hz, H-3′), 2.66 (d, J = 3 Hz, H-4′), 4.22 (q, J = 6.5 Hz, H-5′), 1.22 (d, J = 6.5 Hz, H-6′)

Beispiel 3

Herstellung von ß-Rhodomycinen (Glycosidierungs- und Entblockierungsreaktionen)

7-O-(3′,4′-Bis-(trifluoroacetamido)-2′,3′,4′,6′-tetradesoxy-alpha-L-arabinohexopyranosyl)-10-O-trifluoroacetyl-ß-rhodomycinon (Verbindung 11)

100 mg (0.203 mmol) Verbindung 3 wurden in 20 ml Dichlormethan/Aceton (10:1) aufgenommen und mit 197 mg (2 äq) 3,4-Bis-(trifluoroacetamido)-1-O-p-nitrobenzoyl-2, 3,4,6-tetra-desoxy-alpha,ß-L-arabinohexopyranose und 200 mg Molekularsieb 4 A versetzt. In der auf -30° C abgekühlten Reaktionsmischung wurden 0.1 ml (3 äq) Trifluormethansulfonsäure-trimethylsilylester zugegeben und es wurde 2 Stunden gerührt. Der Reaktionsansatz wurde mit 0.1 ml Triethylamin neutralisiert und abfiltriert. Das Filtrat wurde mit Natriumsulfat verrührt, abfiltriert und in Vakuum eingedampft. Das Rohprodukt wurde säulenchromatographisch über Kieselgel (Elutionsnittel: Dichlormethan/ Aceton 15:1) gereinigt.
Ausbeute: 125 mg (76.6 %)

7-O-(3′-Amino-2′,3′,4′,6′-tetradesoxy-4′-trifluoroacetamido-alpha-L-arabinohexopyranosyl)-ß-rhodomycinon (Verbindung 12)

125 mg (0.155 mmol) Verbindung 11 wurden in 10 ml Chloroform/Methanol gelöst und mit 2 ml 1 n NaOH ver setzt. Nach 2 Stunden wurde die Reaktionsmischung mit 2 ml 1 n HCl neutralisiert und abfiltriert. Das Filtrat wurde in Vakuum eingedampft. Der in Chloroform/Methanol gelöste Rückstand wurde über Natriumsulfat getrocknet und säulenchromatographisch über Kieselgel (Elutionsmittel: Chloroform/Methanol/ Wasser 4:4:1) gereinigt.
Ausbeute: 72 mg (0.76 %)
MS: FAB m/e = 611 $(M+H^+)$

Die Struktur der Verbindung wurde mittels $^1$H-300 MHz-NMR sowie H,H-COSY vor allem die Anwesenheit der 4′-Trifluoroacetamidogruppe aufgeklärt.

7-O-(3′-Azido-2′,3′,4′,6′-tetradesoxy-4′-triifluoroacetamido-alpha-L-lyxohexopyranosyl)-ß-rhodomycinon (Verbindung 13)

115 mg (0.238 mmol) Verbindung 3 wurden in 20 ml Dichlormethan/Aceton 10:1 aufgenommen und mit 300 mg (3 äq) 3- Azido-1-O-p-nitrobenzoyl-2,3,4,6-tetradesoxy-4-trifluoroacetamido-alpha,ß-L-lyxohexopyranose und 250 mg Molekularsieb 4 A versetzt. Der auf -50° C abgekühlten Reaktionsmischung wurden 264 mg (5 äq) Trifluoromethansulfonsäure-trimethylsilylester zugegeben. Nach 2 Stunden wurde der Reaktionsansatz mit 0.16 ml (5 äq) Triethylamin neutralisiert und abfiltriert. Das Filtrat wurde mit wenig n-Butanol vermischt, mit 0.05 n NaOH ausgewaschen, über Natriumsulfat getrocknet und in Vakuum eingedampft. Der Rückstand wurde säulenchromatographisch über Kieselgel (Elutionsmittel: Dichlormethan/Petrolether/Aceton 5:5:1) gereinigt.
Ausbeute: 110 mg (72 %)
MS: FAB m/e = 637 $(M+H^+)$
$^1H$-NMR (270 MHz, $CDCl_3$/MeOD 3:1, delta): 7.82 (dd, H-1), 7.67 (t, H-2), 7.27 (dd, H-3), 5.05 (br.s, H-7), 2.13 (dd, H-8a), 2.07 (dd, H-8b), 5.79 (s, H-10), 1.69 (m, H-13a), 1.76 (m, H-13b), 1.03 (t, H-14), 5.44 (br.s, H-1′), 1.83 (ddd, H-2′ax), 1.94 (dd, H-2′eq), 3.83 (m, H-3′), 4.27 (br.d, H-4′), 4.29 (m, H-5′), 1.15 (d, H-6′)

7-O-(3′,4′-Bis-(trifluoroacetamido)-2′,3′,4′,6′-tetradesoxy-alpha-L-lyxohexopyranosyl)-10-O-trifluoroacetyl-ß-rhodomycinon (Verbindung 14) und 7-O-(3′,4′-Bis-(trifluoroacetamido)-2′,3′,4′,6′-tetradesoxy-alpha-L-lyxohexopyranosyl)-ß-rhodomycinon (Verbindung 15)

100 mg (0.207 mmol) Verbindung 3 wurden in 20 ml Dichlormethan/Aceton 10:1 gelöst, und mit 201 mg (2 äq) 3,4-Bis-(trifluoroacetamido-1-O-p-nitrobenzoyl-2,3,4,6-tetradesoxy-alpha,ß-L-lyxohexopyranose und 200 mg Molekularsieb versetzt. Zu der auf -30° C abgekühlten Reaktionsmischung wurden unter Feuchtigkeitsausschluß 0.1 ml (3 äq) Trimethylsilyltriflat zugegeben. Nach 2 Stunden wurde der Reaktionsansatz mit 0.1 ml Triethylamin versetzt und abfiltriert. Das Filtrat wurde in Vakuum eingedampft, und das resultierende Rohprodukt, das die Verbindung 14 enthält, wurde ohne weitere Reinigung in die nächste Reaktionsstufe eingesetzt. Das Rohprodukt wurde in 10 ml Chloroform/Methanol (3:1) gelöst und mit 1 ml 0.1 n NaOH versetzt. Nach 10 min Reaktionszeit wurde die partielle Entblockierung abgeschlossen. Die Reaktionsmischung wurde mit 1 ml 0.1 n HCl neutralisiert und in Vakuum eingedampft. Der Rückstand wurde säulenchromatographisch über Kieselgel (Elutionsmittel: Dichlormethan/Aceton 10:1) gereinigt.
Ausbeute: 100 mg (83.7 %) Verbindung 15
MS: FAB, m/e = 707 $(M+H^+)$; m/e = 729 $(M+Na^+)$

7-O-(3′,4′-Diamino-2′,3′,4′,6′-tetradesoxy-alpha-L-lyxohexo-pyranosyl)-ß-rhodomycinon (Verbindung 16)

100 mg (0.14 mmol) Verbindung 15 wurden in 10 ml Chloroform/Methanol 3:1 gelöst und mit 2 ml 1 n NaOH versetzt. Nach 2 Stunden Rühren wurde der Reaktionsansatz mit 2 ml 1 n HCl neutralisiert und in Vakuum eingedampft. Das Rohprodukt wurde wie üblich über Natriumsulfat getrocknet und säulenchromatographisch über Kieselgel (Elutionsmittel: Chloroform/Methanol/Wasser 4:4:1) gereinigt.
Ausbeute: 58 mg (80.8 %)
MS: FAB m/e = 515 $(M+H^+)$

7-O-(4′-Azido-2′,3′,4′,6′-tetradesoxy-3′-trifluoroacetamido-alpha-L-arabinohexopyranosyl)-ß-rhodomycinon (Verbindung 17)

1 g (2.07 mmol) Verbindung 3 wurden in 150 ml Dichlormethan/ Aceton (10:1) gelöst und mit 2.6 g (3 äq) 4-Azido-1-O-p-nitrobenzoyl-2′,3′,4′,6′-tetradesoxy-3-trifluoroacetamido-alpha,ß-L-arabinohexopyranose und 2 g Molekularsieb 4 A versetzt. Der auf -50° C abgekühlten Reaktionsmischung wurden unter Schutzgas 1.6 ml (5 äq) Trimethylsilyltriflat zugegeben. Der Reaktionsansatz wurde 2 Stunden gerührt, anschließend mit 1.4 ml (5 äq) Triethylamin verrührt und abfiltriert. Das Filtrat wurde mit 0.01 n NaOH, dann mit Wasser ausgewaschen und in Vakuum eingedampft. Der Rückstand wurde säulenchromatographisch über Kieselgel (Elutionsmittel: Dichlormethan/Aceton 10:1) gereinigt.
Ausbeute: 0.94 g (72 %)

7-O-(3′-O-p-Nitrobenzoyl-2′,4′,6′-tridesoxy-4′-trifluoro-acetamido-alpha-L-lyxohexopyranosyl)-10-O-trifluoroacetyl-ß-rhodomycinon (Verbindung 18)

500 mg (1.03 mmol) Verbindung 3 wurden in 90 ml Dichlormethan/Aceton 10:1 gelöst und mit 1.4 g (2.5 äq) 1,3-Bis-(p-nitrobenzoyl)-2,4,6-tridesoxy-4-trifluoroacetamido-alpha,ß-L-lyxohexopyranose und 1 g Molekularsieb 4 A versetzt. Zu der auf -30° C abgekühlten Reaktionsmischung wurden 1.1 g (5 äq) Trimethylsilyltriflat zugegeben. Nach 2 Stunden Rühren unter Schutzgas wurde der Reaktionsansatz mit 0.7 ml (5 äq) Triethylamin versetzt, abfiltriert und in Vakuum eingedampft. Das Rohprodukt wurde säulenchromatographisch über Kieselgel (Elutionsmittel: Dichlormethan/ Petrolether/Aceton 5:5:1) gereinigt.
Ausbeute: 0.85 g (95 %)

7-O-(4′-Amino-2′,4′,6′-tridesoxy-alpha-L-lyxohexopyranosyl)-ß-rhodomycinon (Verbindung 19)

0.85 g (0.99 mmol) Verbindung 18 wurden in 100 ml Chloroform/Methanol gelöst und mit 10 ml 1 n NaOH versetzt. Nach 2 Stunden wurde der Reaktionsansatz mit 10 ml 1 n HCl neutralisiert und in Vakuum eingedampft. Der Rückstand wurde in Chloroform/Methanol gelöst, mit Natriumsulfat verrührt und abfiltriert. Das Filtrat wurde in Vakuum eingedampft, und das resultierende Rohprodukt wurde säulenchromatographisch über Kieselgel (Elutionsmittel: Chloroform/Methanol/Ammoniak 65:35:1) gereinigt.
Ausbeute: 0.324 g (62 %)
MS: FAB m/e = 516 $(M+H^+)$

Beispiel 4

Zytotoxizität gegen L1210 Leukämiezellen (stem cell assay)

Experimentelles Vorgehen:

Der Test wurde entsprechend der von Hamburger und Salmon beschriebenen Methodik mit den unten beschriebenen Modifikationen durchgeführt.

Konditioniertes Medium wurde durch McCoy 5A Medium ersetzt. Als Folge der hohen Klonierungsrate der L1210-Leukämiezellen in Soft Agar wurde die Anzahl an Tumorzellen pro Platte auf $5 \times 10^2$ reduziert.

Die Zellen wurden mit unterschiedlichen Konzentrationen der Testsubstanz für 1 h bei 37° C inkubiert. Anschließend wurden die Zellen zweimal mit McCoy 5A Medium gewaschen und anschließend in einem Zwei-Schichten-Agar System als obere Schicht entsprechend der Methode von Hamburger und Salmon ausplatiert.

Zusätzliche Parallelexperimente wurden unter Verwendung einer kontinuierlichen Inkubationszeit durchgeführt, wobei unterschiedliche Konzentrationen der Testsubstanz der oberen Agarschicht vor Ausplatieren der Zellen zugemischt wurden.

Die Platten wurden in einem Brutschrank mit 5 % $CO_2$, 20 % $O_2$ und 95 % relativer Luftfeuchte für 5-7 Tage inkubiert. Nach dieser Zeit wurden Kolonien mit einem Durchmesser von 60 µm mit Hilfe eines Invertoskopes gezählt.

Die Ergebnisse wurden angegeben als prozentualer Anteil der Kolonien in behandelten versus unbehandelten Gruppen. Der Variationskoeffizient bei wiederholten Experimenten war kleiner als 15 %.

Resultate:

Die getesteten Verbindungen sind in der Tabelle 1 enthalten.

Aus der Dosis-Wirkungskurve wurde die $IC_{50}$ für die kontinuierliche und einstündige Inkubation bestimmt (Tab. 1).

Diskussion:

Die in der Tabelle zusammengefaßten Zahlen belegen, daß die meisten Substanzen eine sehr hohe Zytotoxizität ($IC_{50}$ kleiner als 0.1 µg/ml) in unterschiedlichen in vitro Testsystemen gegenüber menschlichen und tierischen Tumorzellen aufweisen.

Beispiel 5

Proliferationstest (MTT-Reduktion)

L1210, A 549 oder HT 29 in exponentieller Wachstumsphase werden in einer Zelldichte von 5 x $10^3$ Zellen/ml in RPMI 1640 in einer 96 Well Mikrotest platte für 72 h mit unterschiedlichen Konzentrationen der Testsubstanz bei 37° C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 h Inkubation werden 50 µl einer MTT-Lösung (2,5 mg/ml in Phosphat-gepuffertem Kochsalz) zugegeben. In Gegenwart lebender Zellen wird MTT zu einem dunkelroten unlöslichen Formazanfarbstoff reduziert. Diese Reaktion ist nach 7 h (L1210 Zellen) bzw. nach 24 h (A 549, HT 29 Zellen) beendet, und das überstehende Medium wird sorgfältig abgesaugt. Der unlösliche Farbstoff wird durch Zugabe von 100 µl DMSO aufgelöst und die Extinktion der so entstehenden Lösung anschließend für jedes Well in einem Multiscan Photometer 340 CC der Fa. Flow bei einer Wellenlänge von 492 nm vermessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abtötet, abgelesen werden kann. Für wiederholte Untersuchungen beträgt der Variationskoeffizient weniger als 15 %.

Beispiel 6

Ermittlung der akuten Toxizität

Zur Ermittlung der akuten Toxizität werden BDF1-Mäusen am Tag 0 unterschiedliche Dosen der Testsubstanz, gelöst in 0.5 ml 5 %iger Glucose-Lösung, intraperitoneal injiziert. Kontrollgruppen erhalten lediglich 0.5 ml 5 %ige Glucose-Lösung. Pro Konzentration der Testsubstanz werden 5 Mäuse verwendet. Am Tag 14 wird die Zahl der überlebenden Mäuse ermittelt und daraus nach der Litchfield Wilcoxon Methode die LD5, LD50 und LD95 ermittelt. Die Toxizität (LD50 mg/kg) der hier beschriebenen Verbindungen im Vergleich zu Adriamycin wurde ermittelt.

Beispiel 7

In vivo Wirksamkeit der Rhodomycine gegen L1210 Leukämie der Maus

Methodik:

Ascitesflüssigkeit wird unter sterilen Bedingungen DBA2 Mäusen (weiblich, 18 - 20 g) 7 Tage nach Implantation entnommen. Der Ascites wird dreimal mit PBS gewaschen, gezählt und auf eine Zellzahl von $10^6$ in 0.2 ml PBS eingestellt.

$10^6$ Zellen, suspendiert in 0.2 ml PBS, werden anschließend DBF1 Mäusen (weiblich, 18 - 20 g) intraperitoneal injiziert. 6 Tiere pro Gruppe werden für jede Substanzkonzentration bzw. als Kontrolle eingesetzt.

Ermittlung der antitumoralen Wirksamkeit:

a) Die Tiere werden am Tag 1 und 5 nach Injektion der Testsubstanz gewogen. Gewichtsverlust von

mehr als 20 % am Tag 5 wird als Indikator einer toxischen Substanzwirkung angesehen.

b) Am Ende des Experimentes (Tod aller Tiere oder überlebende Tiere am Tag 60) wird die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wird ausschließlich für im Verlaufe des Experimentes sterbende Tiere bestimmt. Langzeitüberlebende (LTS) werden bei dieser Berechnung nicht berücksichtigt und gesondert aufgeführt.

Aus der mittleren Überlebenszeit ($MST_T$) der behandelten Gruppen sowie der Kontrollgruppen ($MST_c$) wird die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_T}{MST_C} \times 100$$

T/C-Werte größer als 125 % werden als Indikator einer signifikanten antitumoralen Wirksamkeit der Testsubstanz angesehen. Die Dosis, die den größten antitumoralen Effekt (optimale Dosierung) zeigte sowie jeweils eine Dosisstufe oberhalb und unterhalb dieser Dosis wurden ermittelt. Tiere, die am Tag 60 des Experimentes noch leben, werden als Longterm-Survivors getrennt aufgeführt.

Tabelle 1

| | $IC_{50}$ (µg/ml) in vitro | | | | | | L1210 in vivo |
|---|---|---|---|---|---|---|---|
| | SCA | | MTT-Assay | | | | |
| Verbindung Nr. | cont. | 1 h | L1210 | A549 | HT29 | $LD_{50}$ | |
| 8 | | | 0.043 | 0.14 | 0.043 | | |
| 10 | | | 0.039 | 0.095 | 0.033 | | |
| 12 | | 0.44 | 0.03 | 0.027 | 0.022 | | |
| 13 | | 0.29 | 0.10 | 0.10 | 0.20 | | |
| 15 | | 0.43 | 0.037 | 0.059 | 0.10 | | |
| 16 | | 0.069 | 0.01 | 0.04 | 0.069 | | |
| 17 | | 1.4 | 0.33 | 0.52 | 0.34 | | |
| Adriamycin | 0.02 | 0.04 | | | | | |

## Ansprüche

1. Verbindung der allgemeinen Formel I

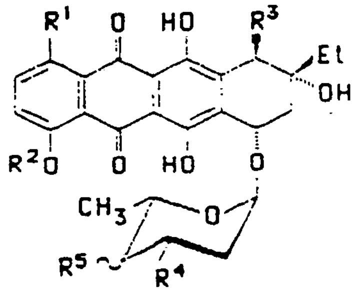

Formel I

worin die Reste folgende Bedeutung haben:
$R^1$ ist ein Wasserstoffatom oder eine Hydroxygruppe,
$R^2$ ist ein Wasserstoffatom oder eine $C_1$-$C_4$-Alkylgruppe,
$R^3$ ist eine Hydroxygruppe, eine O-Acylschutzgruppe oder Methyloxycarbonylgruppe,
$R^4$ ist ein Wasserstoffatom, eine O-Acylschutzgruppe, eine Azidogruppe, eine Amino- oder Trifluoroacetylaminogruppe, eine Di-$C_1$-$C_4$-alkylaminogruppe oder Cyanomethylaminogruppe und
$R^5$ ist eine Azidogruppe, Amino- oder Trifluoroacetylaminogruppe, eine Di-$C_1$-$C_4$-alkylaminogruppe oder Cyanomethylaminogruppe,
wobei Acylschutzgruppe eine Acetyl-, Mono-, Di- oder Trihalogenacetylgruppe- mit Fluor oder Chlor als Halogen oder eine p-Nitrobenzoylgruppe bedeutet, und ihre Salze.

2. Verbindung nach Anspruch 1, worin
$R^1$ H oder OH,
$R^2$ H oder $CH_3$,
$R^3$ OH, $F_3CCOO$, p-$NO_2$-PhCOO oder $COOCH_3$
$R^4$ H, OH,p-$NO_2$-PhCOO, $N_3$, $NH_2$, $NHCOCF_3$, $N(CH_3)_2$ oder $NHCH_2CN$ und
$R^5$ $N_3$, $NH_2$, $NHCOCF_3$, $N(CH_3)_2$ oder $NHCH_2$-CN sind.

3. Verbindung nach Anspruch 1, worin die Reste $R^1$, $R^2$, $R^4$ und $R^5$ die in Anspruch 1 angegebene Bedeutung haben und $R^3$ eine Methyloxycarbonylgruppe ist.

4. Verbindung nach Anspruch 1, worin die Reste $R^1$, $R^2$, $R^4$ und $R^5$ die in Anspruch 1 angegebene Bedeutung haben und $R^3$ eine Hydroxygruppe ist.

5. Verbindung nach Anspruch 1, worin
$R^1$ H oder OH und
$R^2$ eine $C_1$-$C_4$-Alkylgruppe sind und
die Reste $R^3$, $R^4$ und $R^5$ die in Anspruch 1 angegebene Bedeutung haben.

6. Verbindung nach Anspruch 1 als Arzneimittel.

7. Verfahren zur Herstellung einer Verbinding nach Anspruch 1, dadurch gekennzeichnet, daß man eine Aglycon-Verbindung der Formel II

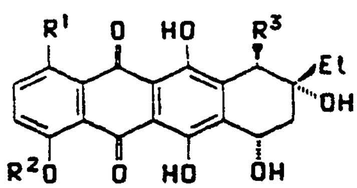

Formel II

worin die Reste
$R^1$ H oder OH
$R^2$ H oder $C_1$-$C_4$-Alkyl und
$R^3$ eine O-Acylschutzgruppe oder $COOCH_3$ bedeuten,
mit einem funktionalisierten Desoxyzucker der Formel III oder IV,

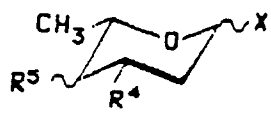

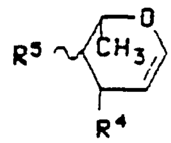

Formel III

Formel IV

worin die Reste

$R^4$ ein Wasserstoffatom, eine O-Acylschutzgruppe, Azidogruppe oder Trifluoroacetylaminogruppe,

$R^5$ eine Azidogruppe oder eine Trifluoroacetylaminogruppe und

X eine Acetyloxy-, p-Nitrobenzoyloxygruppe oder ein Chloratom darstellen,

in Gegenwart eines Katalysators, vorzugsweise eines Trifluoromethansulfonsäure-tri-$C_1$-$C_4$-alkylsilylesters oder dem Silbersalz der Trifluoromethansulfonsäure, zu einem 7-0-Glycosyl-rhodomycinon-Derivat umsetzt, und in dem Produkt die Acylschutzgruppen partiell oder vollständig alkali-hydrolytisch abspaltet und die in dem Produkt vorhandene Azidogruppe hydrogenolytisch in Gegenwart eines Hydrierungskatalysators wie Palladium/Kohle in die Aminogruppe überführt, Kohle in die Aminogruppe überführt, wobei eine Verbindung der Formel I entsteht,

worin die Reste

$R^1$ und $R^2$ unverändert bleiben und

$R^3$ OH oder $COOCH_3$,

$R^4$ ein Wasserstoffatom, eine Hydroxygruppe, eine Acetyloxy-, Trifluoroacetyloxy- oder p-Nitrobenzoyloxygruppe oder eine Azido-, Trifluoroacetylamino- oder Aminogruppe und

$R^5$ eine Azidogruppe, eine Amino- oder Trifluoroacetylaminogruppe darstellen.

Patentanspruch für folgende Vertragsstaaten: ES und GR

1. Verfahren zur Herstellung einer Verbindung nach Formel I

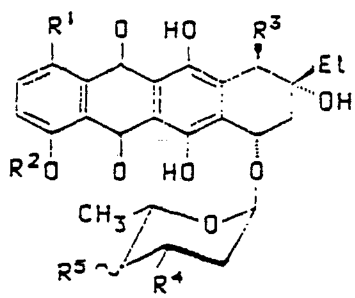

Formel I

worin die Reste folgende Bedeutung haben:

$R^1$ ist ein Wasserstoffatom oder eine Hydroxygruppe,

$R^2$ ist ein Wasserstoffatom oder eine $C_1$-$C_4$-Alkylgruppe,

$R^3$ ist eine Hydroxygruppe, eine O-Acylschutzgruppe oder Methyloxycarbonylgruppe,

$R^4$ ist ein Wasserstoffatom, eine O-Acylschutzgruppe, eine Azidogruppe, eine Amino- oder Trifluoroacetylaminogruppe, eine Di-$C_1$-$C_4$-alkylaminogruppe oder Cyanomethylaminogruppe und

$R^5$ ist eine Azidogruppe, Amino- oder Trifluoroacetylaminogruppe, eine Di-$C_1$-$C_4$-alkylaminogruppe oder Cyanomethylaminogruppe,

wobei Acylschutzgruppe eine Acetyl-, Mono-, Di- oder Trihalogenacetylgruppe- mit Fluor oder Chlor als Halogen oder eine p-Nitrobenzoylgruppe bedeutet, und ihre Salze, **dadurch gekennzeichnet**, daß man eine Aglycon-Verbindung der Formel II

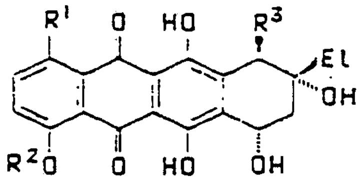

Formel II

worin die Reste
$R^1$ H oder OH
$R^2$ H oder $C_1$-$C_4$-Alkyl und
$R^3$ eine O-Acylschutzgruppe oder $COOCH_3$ bedeuten,
mit einem funktionalisierten Desoxyzucker der Formel III oder IV,

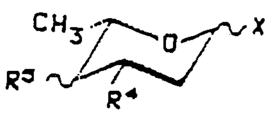

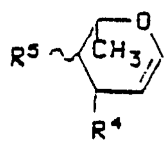

Formel III

Formel IV

worin die Reste
$R^4$ ein Wasserstoffatom, eine O-Acylschutzgruppe, Azidogruppe oder Trifluoroacetylaminogruppe,
$R^5$ eine Azidogruppe oder eine Trifluoroacetylaminogruppe und
X eine Acetyloxy-, p-Nitrobenzoyloxygruppe oder ein Chloratom darstellen,
in Gegenwart eines Katalysators, vorzugsweise eines Trifluoromethansulfonsäure-tri-$C_1$-$C_4$-alkylsilylesters oder dem Silbersalz der Trifluoromethansulfonsäure, zu einem 7-0-Glycosyl-rhodomycinon-Derivat umsetzt, und in dem Produkt die Acylschutzgruppen partiell oder vollständig alkali-hydrolytisch abspaltet und die in dem Produkt vorhandene Azidogruppe hydrogenolytisch in Gegenwart eines Hydrierungskatalysators wie Palladium/Kohle in die Aminogruppe überführt, Kohle in die Aminogruppe überführt, wobei eine Verbindung der Formel I entsteht,
worin die Reste
$R^1$ und $R^2$ unverändert bleiben und
$R^3$ OH oder $COOCH_3$,
$R^4$ ein Wasserstoffatom, eine Hydroxygruppe, eine Acetyloxy-, Trifluoroacetyloxy- oder p-Nitrobenzoyloxygruppe oder eine Azido-, Trifluoroacetylamino-oder Aminogruppe und
$R^5$ eine Azidogruppe, eine Amino- oder Trifluoroacetylaminogruppe darstellen.